# EUROPEAN PATENT SPECIFICATION

(11) **EP 1 690 497 B1**
(45) Date of publication and mention of the grant of the patent: **22.02.2017**
(21) Application number: 04819811.3
(22) Date of filing: 30.11.2004
(51) Int. Cl.: A61B 8/12, A61B 8/06, A61B 1/04, A61B 1/00, A61B 8/13, A61B 8/00, A61B 8/08, G09G 5/14

(54) **ULTRASONOGRAPHIC DEVICE**
ULTRASCHALLGERÄT
DISPOSITIF ECHOGRAPHIQUE

(30) Priority: 02.12.2003 JP 2003403698
(43) Date of publication of application: 16.08.2006
(73) Proprietor: Olympus Corporation, Tokyo 192-8507 (JP)
(72) Inventor: OKUNO, Yoshiyuki, Tokyo 192-8512 (JP); HIBI, Yasushi, Tokyo 192-8512 (JP)
(74) Representative: von Hellfeld, Axel
(86) International application number: PCT/JP2004/017746
(87) International publication number: WO 2005/053539

(56) References cited:
- JP-A- 7 194 596
- JP-A- 7 194 598
- JP-A- 8 117 237
- JP-A- 2003 180 697
- US-A- 4 869 256

## Description

### Technical Field

The present invention relates to an ultrasonic diagnostic apparatus, and more particularly relates to an ultrasonic diagnostic apparatus capable of displaying an endoscopic optical image, an ultrasonic tomographic image, and a blood flow dynamic state image (information) in a body cavity that are generated by an ultrasonic endoscope on a monitor by arbitrarily combining them in response to a request from a surgeon.

### Background Art

An ultrasonic diagnostic apparatus which irradiates an ultrasonic pulse to the interior of a subject, receive the reflected wave of the ultrasonic pulse reflected from tissues in the interior of the subject, performs a predetermined signal processing to the received reflected wave signal, and obtain a tissue tomographic image has been used in the medical field.

Further, in the ultrasonic diagnostic apparatus, in addition to the generation of the tissue tomographic image of the interior of the subject, the Doppler function is used that uses a Doppler effect in which frequencies of an ultrasonic pulse projected to a moving part shift according to moving velocities of the moving part to observe a blood flow dynamic state of the interior of the subject.

In the ultrasonic diagnostic apparatus, by displaying the tomographic image and the blood flow dynamic state image (referred to as color flow image) of the living body tissue in the subject on monitors at the same time, a surgeon and the like can readily understand to which part of the interior of the subject the blood flow dynamic state image (information) being observed belongs.

On the other hand, as an apparatus for obtaining an image of a body cavity of a subject, an endoscope is known whose insertion part is inserted into the interior of the subject and obtains an optical image of the interior of the subject. However, the diagnosis of the interior of the subject using the endoscope is performed based on limited information only about a surface of the interior of the subject in which the endoscope is inserted, and it is not possible to clearly observe a degree of progress to deep part by a tumor, etc. In order to observe the deep part, an ultrasonic endoscope provided with an ultrasonic transducer on a tip end of the endoscope has come into use.

The diagnosis of the interior of the subject can be performed, by the ultrasonic endoscope, by using both images; an endoscopic optical image of the interior of the subject obtained by the observation optical system provided on the tip end of the insertion part to be inserted into the subject, and an ultrasonic tomographic image of deep part of the living body tissue irradiated by the transducer. Further, in the ultrasonic endoscope, by using the above-described Doppler function, it is possible to observe the dynamic state of the blood flow of deep part in the subject in real time.

Thus, by using the ultrasonic endoscope, it is possible to diagnose with images including the endoscopic optical image of the interior of the subject which has color variation, the ultrasonic tomographic image of deep part which is represented by a black and white gradation, and the blood flow dynamic image (information) represented by a color tone based on read and blue.

And now, in general, when displaying an optical image such as an endoscopic image, a color temperature is set according to hue of the interior of a body cavity. On the other hand, in the ultrasonic diagnostic apparatus, a tomographic image in a depth direction of a part to be observed is represented by an image of black and white gradation, and a direction of blood flow and speed are represented by coloring in a Doppler mode.

Although the representing method of the blood flow dynamic state differs, a method of displaying a blood flow signal on a monitor using an endoscopic image and the Doppler effect is proposed in United States Patent No. 6217519. In an ultrasonic endoscope proposed in the United States Patent No. 6217519, the endoscopic optical image and the image of blood flow dynamic state are always displayed on each position on the monitor and sizes of the displayed images are not changed.

Moreover, in general, the ultrasonic endoscope is used in combination with an ultrasonic diagnostic apparatus which generates an ultrasonic tomographic image and an endoscope video processor which generates an endoscopic image, and the ultrasonic tomographic image generated by the ultrasonic diagnostic apparatus and the endoscopic optical image generated by the endoscope video processor are displayed on each monitor respectively.

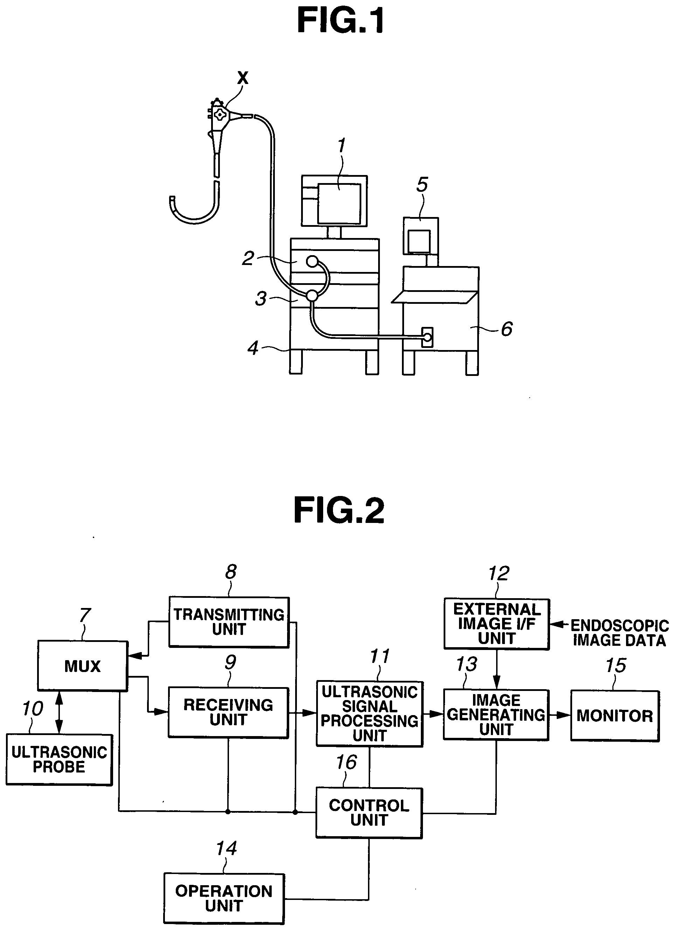

Specifically, as shown in Fig. 1, an endoscopic image monitor 1, an endoscopic video processor 2, and an endoscopic light source 3 are mounted on an endoscopic system trolley 4 and an ultrasonic endoscope X is connected to the endoscopic video processor 2 and an endoscopic light source 3. Further, in order to generate an ultrasonic image, on a trolley different from the endoscopic system trolley 4, an ultrasonic image monitor 5 and an ultrasonic diagnostic apparatus 6 are mounted, and the ultrasonic endoscope X is also connected to the ultrasonic diagnostic apparatus 6.

In the above-described connecting structure, a method of obtaining an endoscopic image by using the ultrasonic endoscope X will be described. The ultrasonic endoscope X irradiates illumination light irradiated from the endoscopic light source 3 from the tip end part of the insertion part. The interior of the subject illuminated by the illumination light is captured by an objective optical system provided in the tip end part of the insertion part and a solid-state image pickup device provided on a focus position of the objective optical system. The captured image signal is processed in the endoscopic video processor 2 with a predetermined signal processing, and displayed on the endoscopic image monitor 1 as an endoscopic image.

Next, a method of obtaining an ultrasonic image by using the ultrasonic endoscope X will be described. The ultrasonic endoscope X transmits an ultrasonic wave by drive controlling the ultrasonic transducer provided on the tip end part of the insertion part by the ultrasonic diagnostic apparatus 6 and receives the returned ultrasonic wave. To the received ultrasonic wave, the predetermined signal processing is performed, and the ultrasonic image is displayed on monitor 5 as an ultrasonic tomographic image.

In the above example, the case in which the ultrasonic endoscope X which has the ultrasonic transducer provided on the tip end part of the insertion part is applied is described. However, an ultrasonic probe which has a built-in ultrasonic transducer inserted from the tip end of the insertion part and protruded by using a forceps channel of an endoscope (not shown) can be applied. Moreover, the shape of the ultrasonic transducer (not shown) can be configured not only by single ultrasonic transducer but also by a plurality of ultrasonic transducers. The shape of the structure is not limited, and may be a fan shape, a linear shape, a radial shape, etc.

In such an ultrasonic endoscope system, when a surgeon inserts the insertion part of the ultrasonic endoscope into the interior of the subject, he performs the insertion while watching the endoscopic optical image displayed on the endoscopic image monitor 1. When the surgeon confirms that the tip end part of the insertion part of the ultrasonic endoscope reaches the observation target part from the endoscopic optical image displayed on the endoscopic image monitor 1, the surgeon performs the generating operation of an ultrasonic tomographic image by driving the ultrasonic transducer by the ultrasonic diagnostic apparatus 6, and performs a diagnostic observation with the tomographic image while shifting his sight line to the ultrasonic tomographic image displayed on the ultrasonic image monitor 5. Accordingly, the surgeon has to turn his eyes from the endoscopic image monitor 1 which displays the endoscopic optical image to the ultrasonic image monitor 5 which displays the ultrasonic tomographic image during the endoscopic observation, and that is a burden to the surgeon in the endoscopic observation.

As described above, if the endoscopic optical image and the ultrasonic tomographic image are displayed on the different monitors, the surgeon is burdened because he has to shift his sight line. Therefore, in order to reduce the surgeon's burden, it is performed that an image output cable of the endoscope video processor 2 and an image output cable of the ultrasonic diagnostic apparatus 6 are connected to the same monitor through a switching device, and by switching the connection by the switching device, the endoscopic optical image and the ultrasonic tomographic image are displayed on the same monitor thus reducing the burden of the surgeon in shifting the sight line. However, since either of the endoscopic optical image or the ultrasonic tomographic image is displayed, in order to confirm whether the part projected by the ultrasonic tomographic image is the expected diagnostic target part or not by using the optical image, the switching operation is necessary. As a result, in the method, the time period necessary for diagnosing becomes long since it is necessary to frequently perform the switching operation to confirm the diagnostic part to be observed.

Meanwhile, as described above, the endoscopic optical image is a colorful image, the ultrasonic tomographic image is a monochrome image represented by the black and white gradation and the blood flow dynamic state image (information) is a color image which varies with respect to each step. In order to diagnose by using these images, it is necessary that each image is displayed on monitors in optimum image quality.

However, as described above, if displaying the endoscopic optical image, the ultrasonic tomographic image, and the blood flow dynamic state image (information) on the same monitor through the switching device, each time displaying each image on the monitor, image quality adjustment is required, and the surgeon is forced to perform a new operation, the image quality adjustment. Further, if the plurality of images is displayed on the monitor at the same time, since the image quality adjustment is performed on the basis of one of these images, image qualities of other images may not be optimized. Moreover, when the ultrasonic tomographic image and the endoscopic optical image are displayed at the same time as described above, when the image quality adjustment is performed on the monitor, either the ultrasonic tomographic image or the endoscopic optical image is prioritized. Accordingly, between the ultrasonic image and the endoscopic image displayed on the monitor, one of the images is optimized, and in some diagnoses, the other image is hard to see for diagnosis, and the surgeon feels difficulty.

The present invention has been made in consideration of the above, and an object of the present invention is to provide an ultrasonic diagnostic apparatus capable of displaying an endoscopic optical image, an ultrasonic tomographic image, and a blood flow dynamic state image on the same monitor, a display position or a size of each image displayed on the monitor can be selected by a surgeon, and each image can be adjusted and displayed in optimal image quality, and providing a circumstance in which the surgeon is able to diagnose by using the displayed image optimized for the surgeon.

US 4,869,458 discloses an endoscope adapted for generating an endoscope image, an ultrasonic probe at a distal end thereof for generating an ultrasonic image, and memory writing control means such that, when it is commanded to view a moving ultrasonic image, a still optical image can be viewed on the same monitor to the side of the moving ultrasonic image in a smaller image display region. Similarly, a still ultrasonic image is displayed to the side of a moving optical image and in a smaller image display region when it is commanded to view the moving optical image.

US 2004/249287 discloses an ultrasonic diagnosis apparatus having an insertable probe and capable of generating ultrasonic and tomographic images. Color data regarding the tomographic image (such as blood flow) are superposed on the tomographic image and output.

### Disclosure of Invention

### Means for Solving the Problem

An ultrasonic diagnostic apparatus according to claim 1 is provided. The ultrasonic diagnostic apparatus may be configured to transmit an ultrasonic wave to the interior of a subject, receive the reflected wave from a living body tissue, obtain an ultrasonic tomographic image and a blood flow dynamic state image in the interior of the subject, while obtain an optical image in the interior of the subject, and display the ultrasonic tomographic image, and the blood flow dynamic
state image, or the endoscopic optical image of the interior of the subject on a monitor. The ultrasonic diagnostic apparatus may have first region display means for displaying the ultrasonic tomographic image or the endoscopic optical image on the display screen of the monitor, second region display means for displaying the endoscopic optical image on a part of the display screen of the monitor, third region display means for displaying the blood flow dynamic state image on the display screen of the monitor, and display image designation means having display image identifying means for identifying image information displayed on the monitor by the first region display means, the second region display means, and the third region display means, designates an image to be displayed on the monitor by each region display means.

The ultrasonic diagnostic apparatus may be configured to transmit an ultrasonic wave to the interior of a subject, receive the reflected wave from a living body tissue, obtain an ultrasonic tomographic image and a blood flow dynamic state image in the interior of the subject, while obtain an optical image in the interior of the subject, and display the ultrasonic tomographic image, and the blood flow dynamic state image, or the endoscopic optical image of the interior of the subject on a monitor. The ultrasonic diagnostic
apparatus has first region display means for displaying the ultrasonic tomographic image or the endoscopic optical image on the display screen of the monitor, second region display means for displaying the endoscopic optical image on a part of the display screen of the monitor, third region display means for superimposing the blood flow dynamic state image on the ultrasonic tomographic image displayed on the display screen of the monitor, and switching means for switching between the ultrasonic tomographic image and the endoscopic optical image displayed on the monitor by the first region display means while switching so as to display the endoscopic optical image by the second region display means and/or the blood flow dynamic state image by the third region display means when the ultrasonic tomographic image is displayed by the first region display means.

### Brief Description of the Drawings

Fig. 1 is a block diagram illustrating an entire structure of a conventional ultrasonic diagnostic apparatus;
Fig. 2 through Fig. 5 relate to a first embodiment of the present invention, in which Fig. 1 is a block diagram illustrating a structure of an ultrasonic diagnostic apparatus according to the first embodiment of the present invention;
Fig. 3 is a block diagram illustrating a structure of an image generating unit in the ultrasonic diagnostic apparatus shown in Fig. 2;
Fig. 4 is a view for explaining examples of displays of images to be displayed on a monitor, in which (A) and (B) are examples in which only a first display region is displayed, (C) is an example in which the first display region and a second display region are displayed, (D) is an example in which the first display region and a third display region are displayed, and (E) is an example in which all of the first display region, the second display region, and the third display region are displayed;
Fig. 5 is a flowchart for explaining operation of the image generating unit;
Fig. 6 through Fig. 9 relate to a second embodiment of the present invention of an ultrasonic diagnostic apparatus, in which Fig. 6 is a block diagram for explaining an ultrasonic diagnostic apparatus;
Fig. 7 is a block diagram illustrating an image combining unit;
Fig. 8 is a view for explaining a CIE color temperature;
Fig. 9 is a scale mapping of a color flow image;
Fig. 10 through Fig. 13 relate to a third embodiment of the present invention, in which Fig. 10 is a block diagram illustrating an ultrasonic diagnostic apparatus;
Fig. 11 is a view for explaining a correspondence of a memory map to a screen according to the third embodiment; and
Fig. 12 is a flowchart for explaining a control method.

### Best Mode for Carrying Out the Invention

Exemplary embodiments of an ultrasonic diagnostic apparatus according to the present invention will be described with reference to the accompanying drawings.

### EMBODIMENT 1

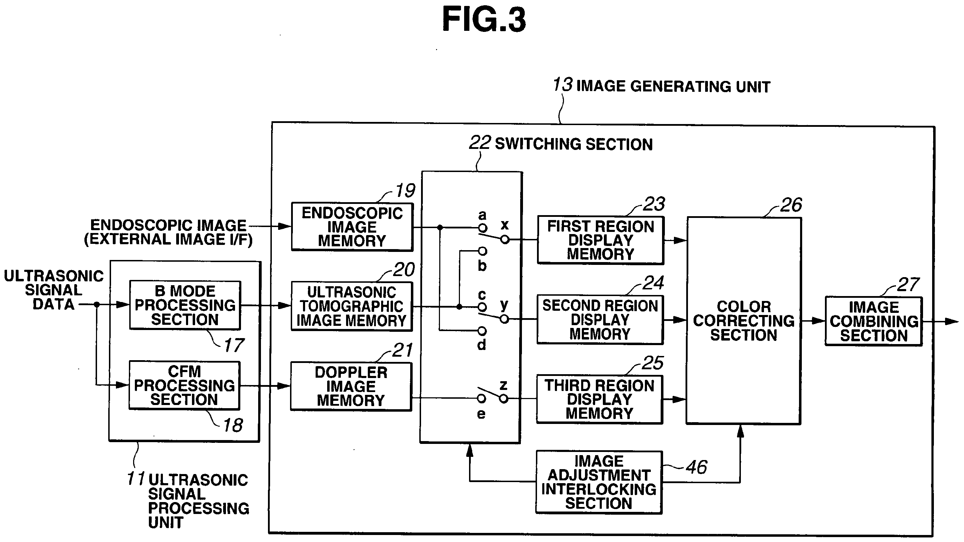

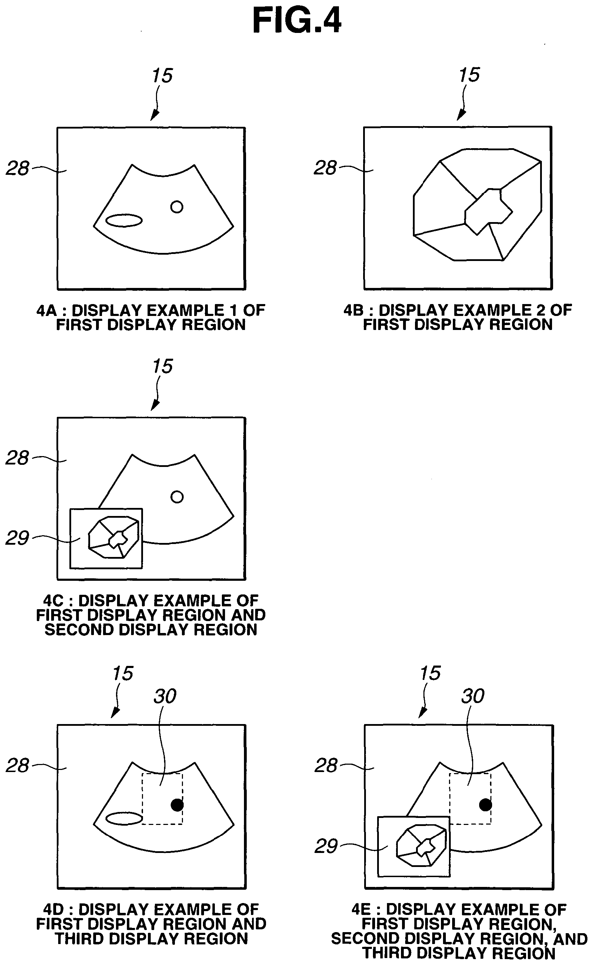

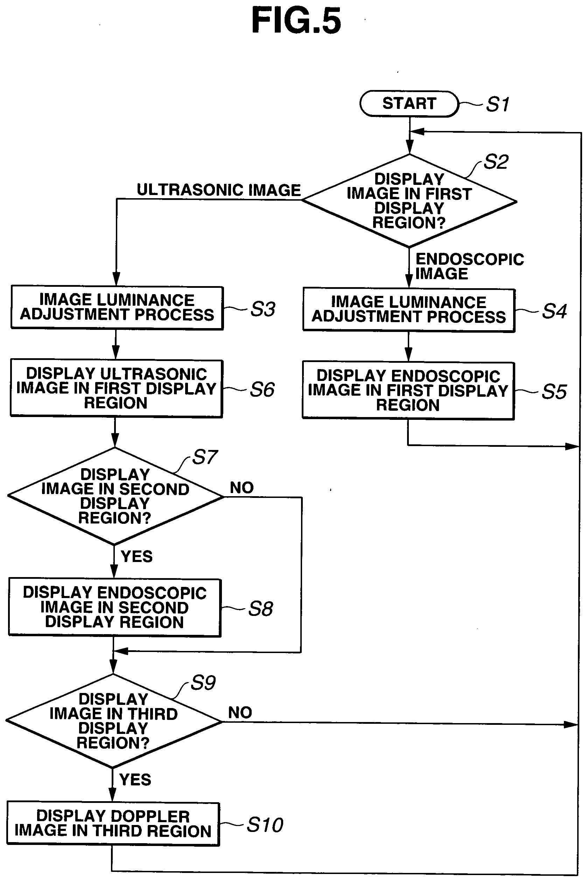

Fig. 2 through Fig. 5 relate to an ultrasonic diagnostic apparatus according to the first embodiment of the present invention, Fig. 2 is a block diagram illustrating a structure of the ultrasonic diagnostic apparatus, Fig. 3 is a block diagram illustrating a structure of an image generating unit in the ultrasonic diagnostic apparatus shown in Fig. 2, Fig. 4 is a view for explaining examples of displays of images to be displayed on a monitor, and Fig. 5 is a flowchart for explaining operation of the image generating unit.

With reference to Fig. 2, the schematic structure of the ultrasonic diagnostic apparatus according to the first embodiment will be described. The ultrasonic diagnostic apparatus has an ultrasonic probe 10, a switching circuit (in the drawing, indicated as MUX, and hereinafter, referred to as MUX) 7, a transmitting unit 8, a receiving unit 9, an ultrasonic signal processing unit 11, an external image interface unit (hereinafter, referred to as external image I/F unit) 12, an image generating unit 13, a monitor 15, a control unit 16, and an operation unit 14.

The ultrasonic probe 10 is either of an ultrasonic endoscope which has a built-in ultrasonic transducer for transmitting and receiving an ultrasonic wave provided on the tip end part of an endoscope insertion part with an objective optical system, or an ultrasonic probe which is inserted into a channel provided to an endoscope insertion part and has an ultrasonic transducer for transmitting and receiving an ultrasonic wave on the tip end part. The ultrasonic probe 10 is inserted into a body cavity, transmits an ultrasonic wave to a living body tissue from the interior of the body cavity, and observes a living body tissue tomogram and blood flow dynamic state by using the reflected ultrasonic wave.

The MUX 7, to the ultrasonic probe 10, switches signals between an ultrasonic transmission driving signal transmitted from the transmitting unit 8 and a reflected ultrasonic wave from the ultrasonic probe 10 to be provided to the receiving unit 9. The transmitting unit 8, through the MUX 7, to the ultrasonic probe 10, generates and provides an ultrasonic transmission driving signal. The receiving unit 9, through the MUX 7, receives the reflected ultrasonic wave from the ultrasonic probe 10, and amplifies it to a signal of a predetermined value.

The ultrasonic signal processing unit 11 performs a predetermined signal processing to the reflected ultrasonic signal which is amplified by the receiving unit 9, and generates an ultrasonic tomographic image data and a blood flow dynamic state information data. The external image I/F unit 12 is an interface which uses a signal captured and generated by a solid-state image pickup device (not shown) provided in the objective optical system on the tip end of the endoscope insertion part, and incorporates endoscopic optical image data generated in an endoscope video processor (not shown)by performing a predetermined signal processing.

The image generating unit 13 generates an image to be displayed on the monitor 15 based on the image data sent from the ultrasonic signal processing unit 11 and the external image I/F unit 12. The monitor 15 displays the image generated in the image generating unit 13.

The control unit 16 controls drive of the MUX 7, the transmitting unit 8, the receiving unit 9, the ultrasonic signal processing unit 11, and the image generating unit 13. The operation unit 14 is used by a surgeon to instruct the control unit 16 to set an image to be processed in the ultrasonic signal processing unit 11, select an image to be displayed on the monitor 15 at the image generating unit 13, adjust image quality, etc.

In the thus structured ultrasonic diagnostic apparatus, by instructions input by the surgeon from the operation unit 14, the drive of the MUX 7, the transmitting unit 8, the receiving unit 9, the ultrasonic signal processing unit 11, and the image generating unit 13 is controlled by the control unit 13. The transmitting unit 8, according to a control by the control unit 16 in response to a B mode which obtains an ultrasonic tomographic image for an ultrasonic diagnosis input by the surgeon from the operation unit 14, or blood flow mode which obtains a blood flow dynamic state by using a Doppler effect of ultrasonic wave, generates an ultrasonic transmission signal corresponding to the input mode, and provides the signal to the ultrasonic probe 10 through the MUX 7. In response to the ultrasonic transmission signal, the ultrasonic probe 10 transmits an ultrasonic wave. The ultrasonic wave reflected by a living body tissue is received by the ultrasonic probe 10 and converted into a reflection ultrasonic signal, and through the MUX 7, output to the receiving unit 9. That is, the MUX 7, under the control of the control unit 16, switches between the provision of the ultrasonic transmission signal provided from the transmitting unit 8 to the ultrasonic probe 10 and the provision of a reflected ultrasonic signal received and generated by the ultrasonic probe 10 to be provided to the receiving unit 9.

The reflected ultrasonic signal provided to the receiving unit 9 is amplified to a predetermined level of signal and output to the ultrasonic signal processing unit 11. The ultrasonic signal processing unit 11 generates an ultrasonic tomographic image data and a blood flow dynamic state information data in response to the B mode which generates the ultrasonic tomographic image from the ultrasonic wave transmitted from the ultrasonic probe 10 based on the ultrasonic transmission signal sent from the transmitting unit 8, or in response to the blood flow mode.

The ultrasonic tomographic image data and the blood flow dynamic state information data generated in the ultrasonic signal processing unit 11 are transferred to the image generating unit 13. The image generating unit 13 uses the endoscopic optical image data sent from the external image I/F unit 12 and the ultrasonic tomographic image data and the blood flow dynamic state information data sent from the ultrasonic signal processing unit 11, according to the display style to display on the monitor 15 input from the operation unit 14 by the surgeon, generates a display image signal of the image to be displayed on the monitor 15 under the control by the control unit 16.

With reference to Fig. 3, structures of the ultrasonic signal processing unit 11 and the image generating unit 13 will be described. The ultrasonic signal processing unit 11 has a B mode processing section 17 which generates the ultrasonic tomographic image data, and a CFM processing section 18 which generates the blood flow image data as described above. The image generating unit 13 has an endoscopic image memory 19, an ultrasonic tomographic image memory 20, a Doppler image memory 21, a switching section 22, a first region display memory 23, a second region display memory 24, a third region display memory 25, a color correcting section 26, an image combining section 27, and an image quality adjustment interlocking section 46.

The endoscopic image memory 16 in the image generating unit 13 stores the endoscopic optical image data provided from the external image I/F unit 12. The ultrasonic tomographic image memory 20 stores the ultrasonic tomographic data generated in the B mode processing section 17 in the ultrasonic signal processing unit 11. The Doppler image memory 21 stores the blood flow dynamic state information data generated in the CFM processing section 18 in the ultrasonic signal processing unit 11.

The switching section 22 has terminals a and d which are connected to the output of the endoscopic image memory 19, terminals b and c which are connected to the output of the ultrasonic tomographic image memory 20, a terminal e which is connected to the output of the Doppler image memory 21, an armature x which switches connection to the first region display memory 23 between the terminal a and the terminal b, an armature y which switches connection to the second region display memory 24 between the terminal c and the terminal d, and an armature z which connects or disconnects the terminal e to the third region display memory 25.

The first to third region display memory 23 temporary stores the image data sent from the endoscopic image memory 19, the ultrasonic tomographic image memory 20, and the Doppler image memory 21 selected in the switching section 22. The first region display memory 23 stores image data in a first display region, which will be described below, to be displayed on the monitor 15, the second region display memory 24 stores image data in a second display region, which will be described below, to be displayed on the monitor 15, and the third region display memory 25 stores image data in a third display region, which will be described below, to be displayed on the monitor 15.

The color correcting section 26 performs a color correcting processing of each image data stored in the first to third region display memories 23 to 25. The specific color correcting processing in the color correcting section 26 will be described in detail.

For example, in an endoscopic optical image, a lumen wall in a body cavity is generally flesh color or white. However, in the lumen wall in the endoscopic optical image, various colors exist, for example, a raised part may be tinged with red, a part of mucous membrane may be tinged with white, and a cauterized part may be tinged with black. In the colors, the hue and the chroma is adjusted to green side so that the red in the raised part is emphasized.

Further, in an ultrasonic tomographic image, a structure of a deep part is represented by the black and white gradation. Generally, a part containing a lot of blood and a wall are represented in white and a lumen such as a blood vessel is represented in black. In order to express various structures by the gradation, variation in the gradation of each color is maintained to be constant and luminance is linearly varied. Further, a correction of a gamma curve according to an input signal level is performed.

In a blood flow dynamic state image generated by an ultrasonic wave, a blood flow direction is identified and represented in read and blue. Further, existence of the blood flow is represented in gradation of orange color. A color correction is performed so that other colors are not mingled in a color tone variation with such red, blue, and orange. Further, the correction of a gamma curve is performed.

In the above description, it is described that image quality corrections of all image data in the first to third region display memories 23 to 25 can be performed in the color correcting section 26. However, the color correction can be performed only to image data of an image to be displayed on the monitor 15. Further, the color correcting processing can be performed to each image data in the first to third region display memories 23 to 25 respectively.

The image combining section 27 converts each image data to which the color correcting processing is performed in the color correcting section 26 into an analog image signal, and combines each analog image signal to generate a display image signal to be displayed on the monitor 15.

As described above, since the drive of the image generating unit 13 is controlled by the control unit 16, at least drive of the switching section 22, the color correcting section 26, and the image combining section 27 is controlled by the control unit 16. It can be possible to provide image data storage detecting means (not shown) which detects that each image data is stored in the endoscopic image memory 19, the ultrasonic tomographic image memory 20, and the Doppler image memory 21, and by image data storage detecting information from the image data storage detecting means, recognition of the storage of the image data is enabled.

Now, with reference to Fig. 4, switching operation of the switching section 22 and the relationship between image data to be stored in the first to third region display memories 23 to 25 and images to be displayed on the monitor 15 will be described. Fig. 4 illustrates a list of images which can be displayed in combination with switched images switched in the switching section 22 shown in Fig. 3. Figs. 4A and 4B are examples in which only the first display region is displayed. In the first display region in Fig. 4A, an ultrasonic diagnostic image and in the first display region in Fig. 4B, an endoscopic image, are shown on full screen of the monitor. These images are displayed in such a manner based on the image data stored in the first display region memory 23 in Fig. 3. Fig. 4C illustrates an example in which the first display region and the second display region are displayed. In the drawing, the second display region is smaller than the first display region, parts of the regions are overlapped with each other, and the second display region is smaller than the first display region. In the drawing, an example in which an ultrasonic tomographic image is displayed in the first display region and an endoscopic image is displayed in the second display region, is shown. The image data stored in the first display region memory 23 in Fig. 3 is displayed on full screen of the monitor and the data stored in the second display region memory 24 is displayed on a limited region of the screen of the monitor. Further, Fig. 4D illustrates an example in which the first display region and the third display region are displayed. In the drawing, an ultrasonic tomographic image is displayed in the first display region and a CFM image is displayed in the third display region. The image data stored in the first display region memory 23 in Fig. 3 is displayed on full screen of the monitor and the data stored in the third display region memory 25 is displayed on a limited region on the first display region. Fig. 4E illustrates an example in which all of the first display region, the second display region, and the third display region are displayed.

The monitor 15 has a first display region 28 in which an image is displayed on full screen, a second display region 29 in which a reduced image is displayed on a part of the screen, and a third display region 30 in which an image is displayed by superimposing on the first display region displayed on the screen.

The image data of the image to be displayed on the first display region 28 in the monitor 15 is stored in the first display region memory 23, the image data of the image to be displayed on the second display region 29 in the monitor 15 is stored in the second display region memory 24, and the image data of the image to be displayed on the third display region 30 in the monitor 15 is stored in the third display region memory 25.

If the armature x in the switching section 22 is connected to the terminal b, the ultrasonic tomographic image data stored in the ultrasonic tomographic image memory 20 is output to the first display region memory 23 and temporarily stored. To the ultrasonic tomographic image data stored in the first display region memory 23, a color correcting processing is performed in the color correcting section 26, the data is converted into an analog image signal and output to the monitor 15 in the image combining section 27, and as shown in Fig. 4A, displayed on the first display region 28 of the monitor 15 as an ultrasonic tomographic image.

If the armature x in the switching section 22 is connected to the terminal a, the endoscopic optical image data stored in the endoscopic image memory 19 is output to the first display region memory 23 and temporarily stored. To the endoscopic optical image data stored in the first display region memory 23, a color correcting processing is performed in the color correcting section 26, the data is converted into an analog image signal and output to the monitor 15 in the image combining section 27, and as shown in Fig. 4B, displayed on the first display region 28 of the monitor 15 as an endoscopic optical image.

If the armature x in the switching section 22 is connected to the terminal b, and the armature y is connected to the terminal d, through the armature x from the terminal b, the ultrasonic tomographic image data stored in the ultrasonic tomographic image memory 20 is output to the first display region memory 23 and temporarily stored and through the armature y from the terminal d, the endoscopic optical image data stored in the endoscopic image memory 19 is output to the second display region memory 24 and temporarily stored. To the ultrasonic tomographic image data stored in the first display region memory 23 and the endoscopic optical image data stored in the second region display memory 24, color correcting processings are performed in the color correcting section 26, the data is converted into an analog image signal, a combined image signal composed of the first display region 28 and the second display region 29 is generated, and output to the monitor 15 in the image combining section 27, and as shown in Fig. 4C, displayed on the first display region 28 as an endoscopic tomographic image and on the second display region 29 as an endoscopic optical image. Further, if the armature x in the switching section 22 is connected to the terminal a, and the armature y is connected to the terminal c, image data to be stored in the first display region memory 23 and the second display region memory 24 are replaced with an endoscopic optical image data and un ultrasonic tomographic image data, on the monitor 15, the relationship is opposite to that shown in Fig. 4C, the endoscopic optical image can be displayed on the first display region 28 and the ultrasonic tomographic image can be displayed on the second display region 29.

If the armature x in the switching section 22 is connected to the terminal b, and the armature z is connected to the terminal e, through the armature x from the terminal b, the ultrasonic tomographic image data stored in the ultrasonic tomographic image memory 20 is output to the first display region memory 23 and temporarily stored and through the armature z from the terminal e, the blood flow dynamic state information data stored in the Doppler image memory 21 is output to the third display region memory 25 and temporarily stored. To the ultrasonic tomographic image data stored in the first display region memory 23 and the blood flow dynamic state information data stored in the third region display memory 25, color correcting processings are performed in the color correcting section 26, the data is converted into an analog image signal, a combined image signal composed of the first display region 28 and the third display region 30 is generated, and output to the monitor 15 in the image combining section 27, and as shown in Fig. 4D, displayed on the first display region 28 as an endoscopic tomographic image and on the third display region 30 as a blood flow dynamic state information data.

If the armature x in the switching section 22 is connected to the terminal b, the armature y is connected to the terminal d, and the armature z is connected to the terminal e, through the armature x from the terminal b, the ultrasonic tomographic image data stored in the ultrasonic tomographic image memory 20 is output to the first display region memory 23 and temporarily stored, through the armature y from the terminal c, the endoscopic optical image data stored in the endoscopic image memory 19 is output to the second display region memory 24 and temporarily stored, and through the armature z from the terminal e, the blood flow dynamic state information data stored in the Doppler image memory 21 is output to the third display region memory 25 and temporarily stored. To the ultrasonic tomographic image data stored in the first display region memory 23, the endoscopic optical image data stored in the second display region memory 24, and the blood flow dynamic state information data stored in the third region display memory 25, color correcting processings are performed in the color correcting section 26, the data is converted into an analog image signal, a combined image signal composed of the first display region 28, the second display region 29, and the third display region 30 is generated, and output to the monitor 15 in the image combining section 27, and as shown in Fig. 4E, displayed on the first display region 28 as an endoscopic tomographic image, on the second display region 29 as an endoscopic optical image, and on the third display region 30 as a blood flow dynamic state information.

With reference to Fig. 5, the drive controlling operation for switching images to be displayed on the monitor 15 in the image generating unit 11 by the control unit 16 will be described. As an example of the operation, a case in which an ultrasonic tomographic image and an endoscopic optical image are switched and displayed on the first display region 28 in the monitor 15, an endoscopic optical image is displayed on the second display region 29 in the monitor 15, and a blood flow dynamic state information is displayed on the third display region 30 in the monitor 15, will be described.

When the control unit 15 drives and starts the image generating unit 11 (step S1), the control unit 16 determines whether an image to be displayed on the first display region 28 in the monitor 15 input and instructed by the operation unit 14 is an endoscopic optical image or an ultrasonic tomographic image at step S2.

As a result of the determination at step S2, if it is determined that an input of the endoscopic optical image is instructed, at step S4, the control unit 16 connects the armature x in the switching section 22 to the terminal a, outputs the endoscopic optical image data in the endoscopic image memory 19 to the first region display memory 23 and stores the data, and controls the drive of the color correcting section 26 so as to perform a color correcting processing for endoscopic optical image such as a image luminance, and hue correction to the endoscopic optical image data stored in the first region display memory 23. Then, at step S5, the control unit 16 controls the drive of the image combining section 27, converts the endoscopic optical image to which the color correcting processing is performed into an analog image signal, as shown in Fig. 4B, displays the endoscopic optical image on the first display region 28 in the monitor 15, and return to step S2.

At step S2, if it is determined that an input of the ultrasonic tomographic image is instructed, at step S3, the control unit 16 connects the armature x in the switching section 22 to the terminal b, outputs the ultrasonic tomographic image data in the ultrasonic tomographic image memory 20 to the first region display memory 23 and stores the data, and controls the drive of the color correcting section 16 so as to perform a correction process of black and white gradation for ultrasonic tomographic image to the ultrasonic tomographic image data stored in the first region display memory 23. Then, at step S6, the control unit 16 controls the drive of the image combining section 27, converts the ultrasonic tomographic image data to which the correction process is performed in step S3 into an analog image signal, as shown in Fig. 4A, displays the ultrasonic tomographic image on the first display region 28 in the monitor 15.

At step S7, the control unit 16 determines whether an input instruction of displaying an image on the second display region 29 in the monitor 15 is performed from the operation unit 14 or not. At step S7, if it is determined that an input instruction of not displaying the image on the second display region 29 is performed, subsequent steps after step S9 are performed. At step S7, if it is determined that an input instruction of displaying the image on the second display region 29 is performed, at step S8, the control unit 16 connects the armature y in the switching section 22 to the terminal d, outputs the endoscopic optical image data in the endoscopic image memory 19 to the second region display memory 24 and stores the data, and controls the drive of the color correcting section 26 so as to perform a color correcting processing for endoscopic optical image to the endoscopic optical image data stored in the second region display memory 24, and controls the drive of the image combining section 27, converts the endoscopic optical image into an analog image signal, as shown in Fig. 4C, displays the endoscopic optical image on the second display region 29 in the monitor 15.

Then, at step S9, the control unit control unit 16 determines whether an input instruction of displaying an image on the third display region 30 in the monitor 15 is performed from the operation unit 14 or not. At step S9, if it is determined that an input instruction of not displaying the image on the third display region 30 is performed, the control unit 16 returns to step S2. At step S9, if it is determined that an input instruction of displaying the image on the third display region 30 is performed, at step S10, the control unit 16 connects the armature z in the switching section 22 to the terminal e, outputs the blood flow dynamic state information data in the Doppler image memory 21 to the third region display memory 25 and stores the data, and controls the drive of the color correcting section 26 so as to perform a color correcting processing for blood flow dynamic state information to the blood flow dynamic state information data stored in the third region display memory 25, and controls the drive of the image combining section 27, converts the data into an analog image signal, as shown in Fig. 4E, displays the endoscopic optical image on the third display region 30 in the monitor 15.

That is, as shown in Fig. 4(E), by the process from step S3 through step S10, it is possible to display the ultrasonic tomographic image on the first display region 28, the endoscopic optical image on the second display region 29, and the blood flow dynamic state information on the third display region at the same time, and the luminance, hue, gradation, etc. of these images are corrected and displayed in optimum conditions.

The second region display memory 24, in order to display a reduced endoscopic optical image, has a compression section (not shown) in the memory. Further, the third region display memory 25, if an amount of image data is less than the image display region, can have a correcting section for increasing the number of data.

Further, the color correcting section 26, depending on the type of each image data, can have a memory in which color correction parameter data which stores the correction data is stored. The color correction parameter data is synchronized with the input of the input instruction of switching image displays into the image generating unit 13 by the operation unit 14 through the control unit 16, read out at an appropriate timing and applied so that the data is applied to the image data to be processed in the color correcting section 26.

Further, on the screen on which the combination of the first display region and the second display region is displayed as shown in fig. 4C, the position of the second display region is not limited to the position shown in the drawing, the position can be moved right, left, up, and down by an instruction from the operation unit 14. Further, on the screen on which the combination of the first display region, the second display region and the third display region is displayed as shown in fig. 4E, similarly, the positions of the second display region and the third display region are not limited to the position shown in the drawing, the positions can be respectively moved right, left, up, and down by an instruction from the operation unit 14. Further, if the second display region and the third display region are overlapped, by a setting from the operation unit 14, by a control by the image combining section 27, the positions can be automatically moved to positions where they do not overlap.

Further, the color correction parameter data is synchronized with the input of the input instruction of switching image displays into the image generating unit 13 in Fig. 2 by the operation unit 14 in Fig. 2 through the control unit 16 in Fig. 2, and switched at an appropriate timing so that the data is applied to the image data to be processed in the color correcting section 26 in Fig. 3. Then, interlocking with the switching section 22, in response to the switched image, an image quality adjustment interlocking section 46 can be provided so that the correction data is switched in the color correcting section 26, and interlocking with the switching of the images, parameters of the color correction can be switched.

### EMBODIMENT 2

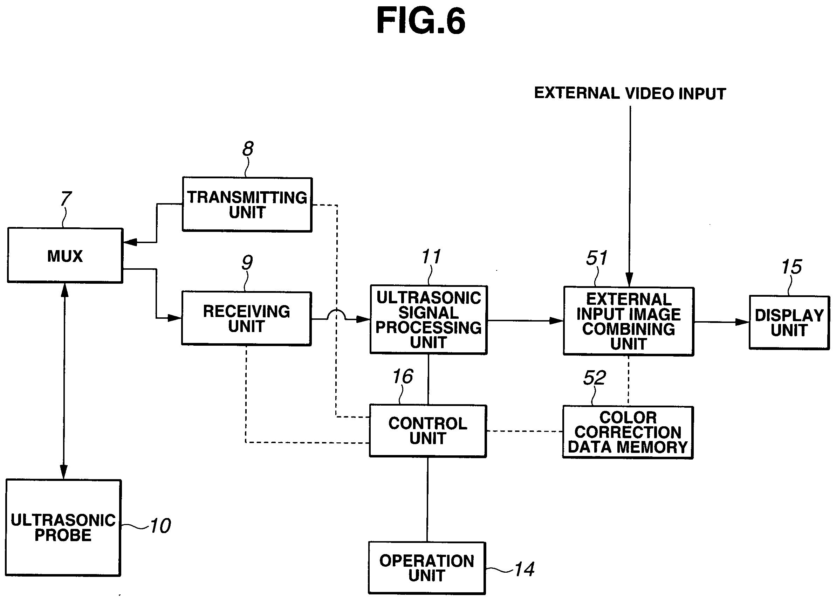

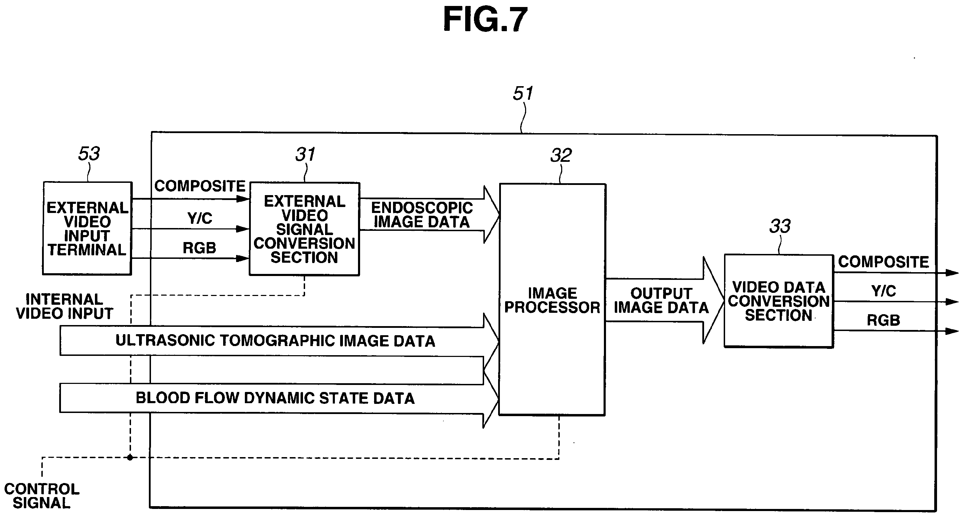

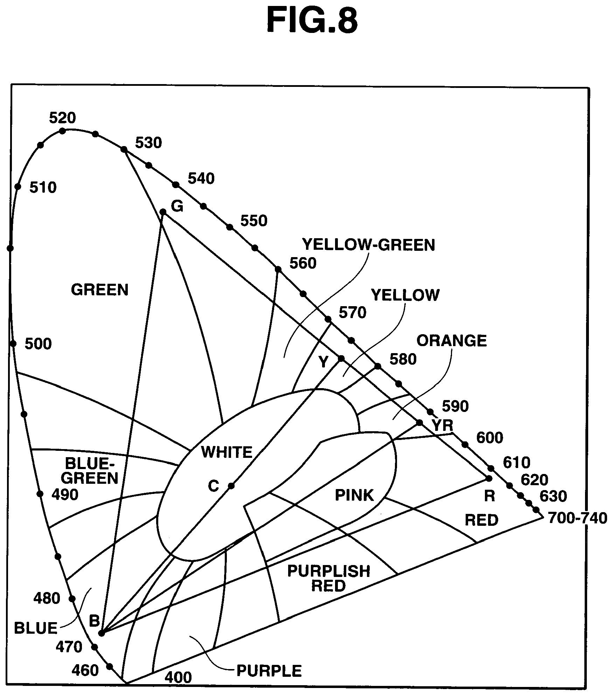

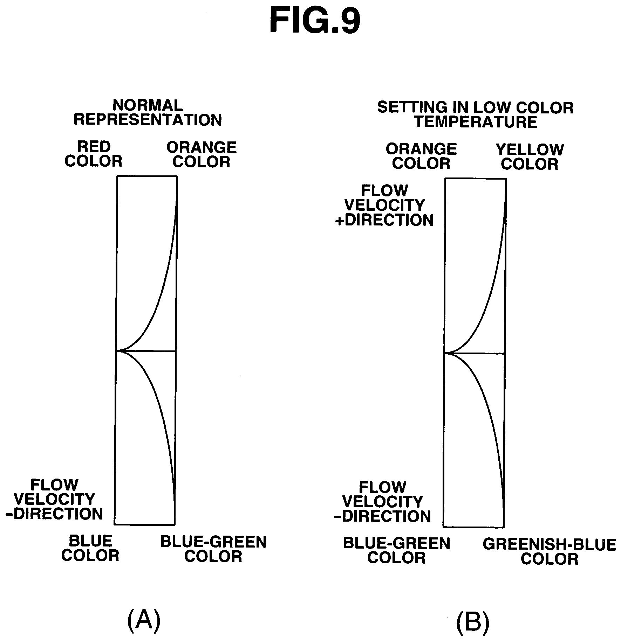

Fig. 6 through Fig. 9 illustrate an ultrasonic diagnostic apparatus according to a second embodiment of the present invention. Fig. 6 is a block diagram for explaining the ultrasonic diagnostic apparatus, Fig. 7 is a block diagram illustrating an image combining section, Fig. 8 is a view for explaining a CIF color temperature, and Fig. 9 is a scale mapping of a color flow image.

The MUX (switching circuit) 7, the transmitting unit 8, the receiving unit 9, the ultrasonic probe 10, the ultrasonic signal processing unit 11, the operation unit 14, the monitor 15, and the control unit 16 are similar to those shown in Fig. 2. Added and modified parts compared with the structure in Fig. 1 are the image combining section and the color correction data memory. In the second embodiment, an image combining unit with external input 51 and a color correction data memory 52 are modified and added.

As shown in Fig. 6, in the embodiment, the operation is similar to that shown in the embodiment 1 in which by an instruction by the operation unit 14, through the control unit 16, an ultrasonic wave is transmitted and received from the ultrasonic probe 10 by using the transmitting unit 8, and the receiving unit 9, the obtained ultrasonic echo data is processed in the ultrasonic signal processing unit 11 and generated as an ultrasonic tomographic image data and a blood flow dynamic state information data. Then, the ultrasonic tomographic image data and the blood flow dynamic state information data output from the ultrasonic signal processing unit 11 should be digital video data in compliance with the ITU REC656 standard etc.

The ultrasonic tomographic image data and the blood flow dynamic state information data obtained from the ultrasonic signal processing unit 11 is taken in the image combining unit with external input 51. On the other hand, as shown in Fig. 7, the image combining unit with external input 51 takes in an endoscopic image signal from an external image input terminal 53. The endoscopic image signal, the ultrasonic tomographic image data, and the blood flow dynamic state information data taken in the image combining unit with external input 51 is displayed on the monitor 15 in the combinations shown in Figs. 4A through 4E.

With reference to Fig. 7, the image combining unit with external input 51 will be described.

The image combining unit with external input 51 shown in Fig. 7 has an external video input terminal 53, an external video signal conversion section 31, an image processor 32, and a video data conversion section 33.

An endoscopic video is input from the external video input terminal 53 which has a plurality of kinds of terminals for video signal, the video signal is taken in the external video signal conversion section 31 in a plurality of kinds of video signal formats, converted into digital data in compliance with the ITU REC656 standard etc. in the external video signal conversion section 31, and output. The converted external video data is input into the image processor 33.

The ultrasonic tomographic image data, and the blood flow dynamic state information data shown in Fig. 6 output form the ultrasonic signal processing unit 11 is input into the image processor 32 without change. In the image processor 32, a process is performed so that the output image data shown in region (A) through (E) in Fig. 4 can be obtained with the external video data which is an endoscopic image, the ultrasonic tomographic image data, and the blood flow dynamic state data.

The image processor 32 has a function to correct effect of a color temperature of the monitor. The process will be described. If the color temperature of the monitor is low, it is generally known that the displayed image has a tinge of red. In Fig. 8, a general CIE color temperature is shown. From Fig. 8, it is understood that if the color temperature is set to be low, a color shifts to the side of red, and therefore, the color is set to be a bright color so that the color of the image data to be output for display shifts to an expected color when the image data is displayed.

In a case of a color scale which maps a blood flow state by ultrasonic Doppler will be described. Fig. 9 illustrates an example of color scale in Doppler. If a color temperature of the monitor is set to be low, and a flow velocity is positive as shown in Fig. 9A, a red color and an orange color are set, however, as shown in Fig. 9B, an orange color and a yellow color are set. As a result, the result displayed on the monitor becomes shown in Fig. 9A, even if an endoscopic image is combined, the blood flow state is displayed in a color expected by the surgeon.

The image processor 32 has the function of adjusting a hue, chroma, etc described in the description of the embodiment 1, and the function of setting images to be displayed in the display regions shown in Fig. 4, and the description of these functions will be omitted. Various parameters used in the above-described image adjustment function are stored in the color correction data memory 52 shown in Fig. 6.

Returning to the description of Fig. 7, as a result of the above-described process, the image output data obtained in the image processor 32 is input in the video data conversion section 33, converted into a plurality of kinds of video signal formats, for example, a composite signal, Y/C signal, or RGB signal, output from the external input image combining unit 51, and the image is displayed on the display unit 15.

### EMBODIMENT 3

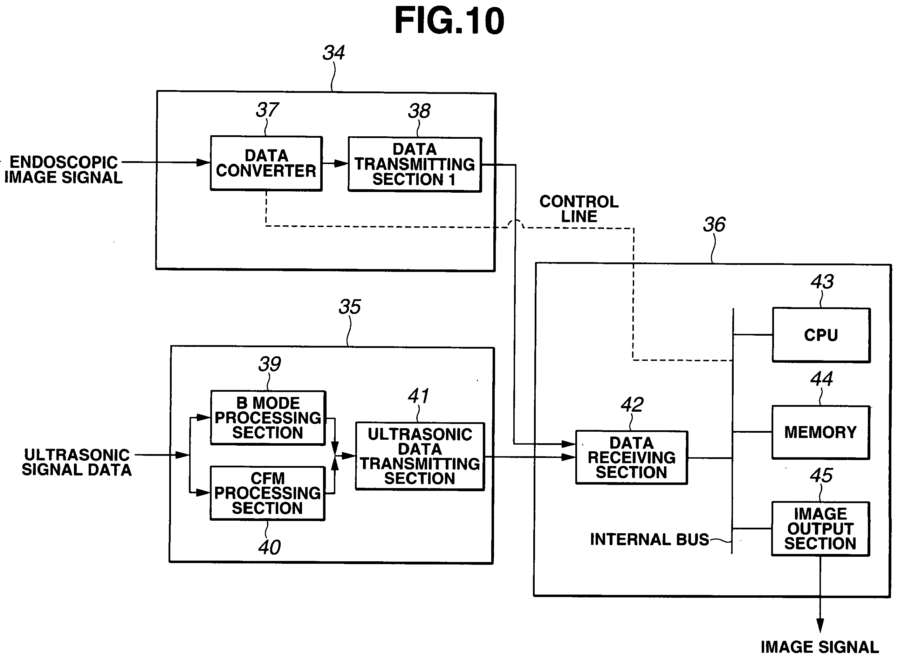

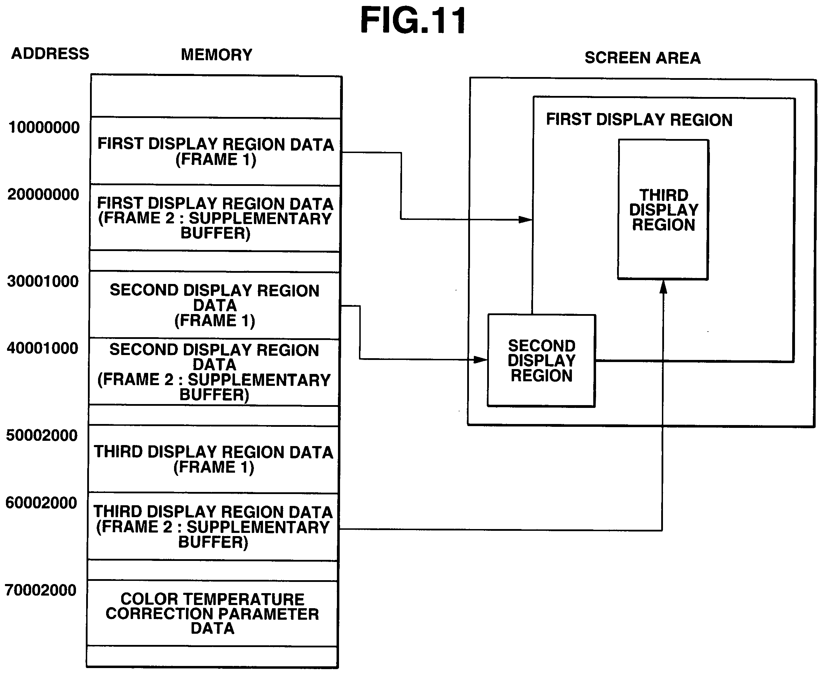

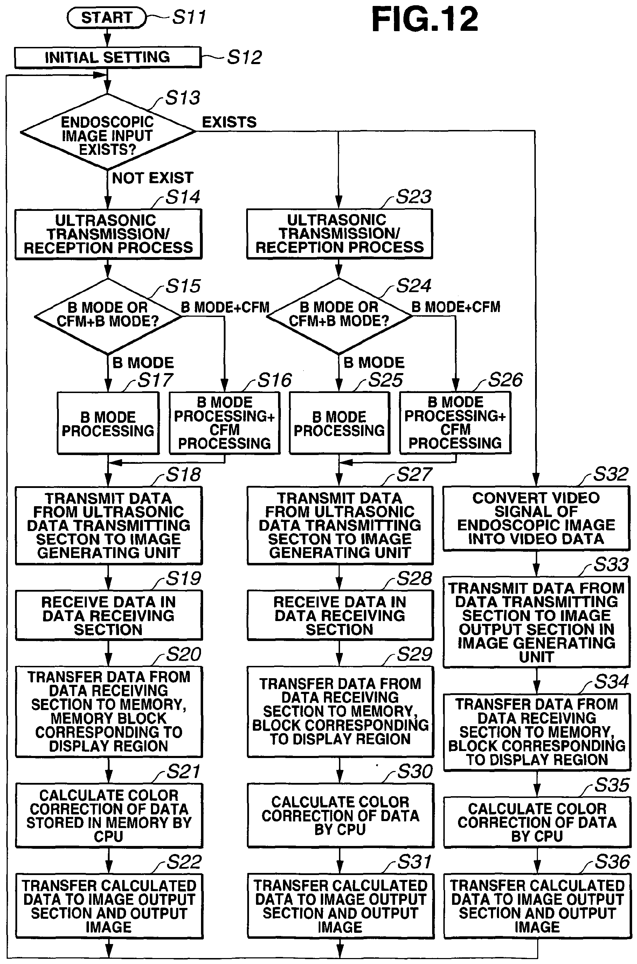

Fig. 10 through Fig. 13 relates to a third embodiment of the present invention. Fig. 10 is a block diagram illustrating an ultrasonic diagnostic apparatus, Fig. 11 is a view for explaining a correspondence of a memory map to a screen, and Fig. 12 is a flowchart for explaining a control method.

The ultrasonic diagnostic apparatus in the embodiment has an external image I/F 34, an ultrasonic signal processing unit 35, and an image generating unit 36. The external image I/F 34 has a data converter 37 and data transmitting section 38. On the other hand, the ultrasonic signal processing unit 35 has a B mode processing section 39, a CFM processing section 40, and an ultrasonic data transmitting section 41. The image generating section 36 has a data receiving section 42, a CPU 43, a memory 44, and an image output section 45.

In Fig. 10, the description of the operation in which an ultrasonic signal is transmitted and received in the ultrasonic probe and the received ultrasonic signal is detected, and the description of the means of controlling the transmission and reception in the Doppler and B mode are omitted.

As shown in Fig. 10, an endoscopic image signal is input in the external image I/F 34 and converted into a video data in the data converter 37. The converted video data is input from the data transmitting section 38 into the data receiving section 42, and stored in the memory 44 through an internal bus. On the other hand, the ultrasonic data which is obtained by transmitting and receiving the ultrasonic wave and the received ultrasonic signal is detected, is input into the ultrasonic signal processing unit 35, an ultrasonic tomographic image data is transferred to the B mode processing section 39, and a Doppler data is transferred to the CFM processing unit, the ultrasonic tomographic image data is output from the B mode processing section 39, and a blood flow dynamic state data is output from the CFM processing unit, and the data is transferred to the ultrasonic data transmitting section 41. The ultrasonic tomographic image data and the blood flow dynamic state data from the ultrasonic data transmitting section 41 is received in the data receiving section 42 of the image generating unit 36, and stored in the memory 44 through the internal bus.

Fig. 11 illustrates a state of the data stored in the memory 44. As shown in Fig. 11, corresponding to the display regions shown in Fig. 4, data areas are divided. For example, if the data is displayed in the first display region, the data is stored in first display region data at an address 10000000. If the next frame data is input before the image data stored in the address 10000000 has not read out yet, the data is stored at address 20000000, and set a flag which means the storage. Accordingly, when the data in the next frame is read out, a program which processes images can be operated in response to the flag. As a result, switching of images in each display region can be possible by switching the stored data, the switching of the memories by the hardware described in the first embodiment becomes unnecessary. Thus, the CPU understands which image is to be displayed in which region. Since a color correction is performed in the CPU, the switching of correction processes interlocking with the image displays is controlled by the CPU. The embodiment 3 is an improved embodiment of the first embodiment.

To the data stored in the memory 44 is, by the control of the CPU 43, various image processing is performed and output from the image processing section 45 as a video signal.

The control method according to this embodiment is shown in a flowchart shown in Fig. 12.

In Fig. 12, at step S12, an initial setting is performed, at step S13, whether an endoscopic image is input or not is determined. If the endoscopic image is input, moves to an ultrasonic transmission/reception processing step S23 and S32 which converts a video signal of the endoscopic image into video data, and if only an ultrasonic image is input, moves to an ultrasonic transmission/reception processing 14.

If the endoscopic image is not input, an ultrasonic transmission/reception process is performed at step S14, and it is determined at step S15 whether an image to be displayed on the monitor is only an ultrasonic tomographic image or a combination of the ultrasonic tomographic image and a blood flow image. If it is determined that only the ultrasonic tomographic image is to be displayed, only a B mode processing is performed at step S17, and if the combination of the ultrasonic tomographic image and a blood flow image is to be displayed, a B mode processing and a CFM processing are performed at step S16. These results of the processes are sent out from the ultrasonic data transmitting section to the image generating unit at step S18, and the data is received in the data receiving section at step S19. The received data is transferred from the data receiving section to a memory block corresponding to the display region shown in Fig. 11 in the memory at step S20, and a color correcting processing of the data stored in the memory by the CPU is performed at step S21. Parameters used in the step S21 are stored in the memory in advance as shown in Fig. 11. Then, the data calculated at step S22 is transferred to the image processing section and output.

If it is determined that the endoscopic image is input at step S13, an ultrasonic transmission/reception process is performed at step S23, while the endoscopic image is converted into video data at step S32. Since processes performed in steps 24 to 31 are similar to those performed in steps S15 to S22, the description of steps 24 to 31 is omitted and processes performed in steps after step S32 will be described. At step S32, the video signal of the endoscopic image is converted in video data, and the converted video data is transmitted from the data transmitting section to the image output section in the image generating unit at step S33. The data is transferred from the data receiving section to the memory block corresponding to the display region shown in Fig. 11 in the memory at step S34, and a color correcting processing of the data stored in the memory by the correction data stored in the memory is performed by the CPU at step S35. The data calculated at step S36 is transferred to the image output section and the image is output. The output ultrasonic tomographic image data and the endoscopic image data is combined in the image combining section.

In the above-described flow, the color correction of the various image data is performed in the CPU 40. However, depending on processing power of the CPU 40, the above-described process of the flow can be flexibly allocated. Further, since the ultrasonic diagnostic apparatus is configured to perform the color matching between the ultrasonic images and other images, images to be combined can be a CT image or a three-dimensional navigation image.

As described above, by the ultrasonic diagnostic apparatus according to the embodiments of the present invention, it can be possible for the surgeon to diagnose and observe the part to be observed from each image displayed on one screen of the monitor 15, and efficiency in the diagnosis and observation of the part to be observed by the ultrasonic endoscope can be increased. As described above, in the ultrasonic diagnostic apparatus according to the embodiments of the present invention, since the endoscopic optical image, the ultrasonic tomographic image, and the blood flow dynamic state information can be displayed on the same monitor in optimum image quality, and the selection such as the selection of images to be displayed on the monitor, combinations, display positions, sizes and the like, can be arbitrarily set, the operational burden on the surgeon in the ultrasonic diagnosis can be reduced, and efficiency in the ultrasonic diagnosis can be

In the ultrasonic diagnostic apparatus according to the present invention, as described above, since it is possible to display an endoscopic optical image, an ultrasonic tomographic image, and a blood flow dynamic state image on the same monitor in a combination necessary for a surgeon in diagnosis, by reducing movement of shifting sight line in diagnosis, the burden in the diagnosis can be reduced.

Further, in the ultrasonic diagnostic apparatus according to the present invention, when switching an ultrasonic tomographic image and an endoscopic optical image, it is possible to realize optimum representation of gradation in each image quality display mode since image qualities such as a luminance of image, or an adjustment of chroma suitable for each image are adjusted.

## Claims

1. An ultrasonic diagnostic apparatus configured to transmit an ultrasonic wave to the interior of a subject, receive the reflected wave from a living body tissue, obtain an ultrasonic tomographic image in the interior of the subject, while obtaining an endoscopic optical image in the interior of the subject, and to display at least one of the ultrasonic tomographic image and the endoscopic optical image on a monitor (15), the ultrasonic diagnostic apparatus comprising:
first region display means (23) for displaying one of the ultrasonic tomographic image or the endoscopic optical image in a first display region (28) of the display screen of the monitor (15);
second region display means (24) for displaying the endoscopic optical image in a second display region (29) on a part of the display screen of the monitor (15);
**characterized in that**
the ultrasonic diagnostic apparatus is further configured to obtain and display a blood flow dynamic state image and further comprises:
third region display means (25) for superimposing the blood flow dynamic state image in a third display region (30) on the ultrasonic tomographic image displayed in the first display region (29) on the display screen of the monitor (15);
switching means (22) for switching between the ultrasonic tomographic image and the endoscopic optical image displayed on the monitor (15) by the first region display means (23) while switching so as to display the endoscopic optical image by the second region display means (24) and/or the blood flow dynamic state image by the third region display means (25) when the ultrasonic tomographic image is displayed by the first region display means (23);
image quality adjusting means (26) for adjusting luminance and chroma of an image displayed on the monitor (15) by the first region display means (23), the second region display means (24), and the third region display means (25) respectively;
image quality adjustment data storing means (52) for storing adjustment contents of the image quality adjusting means (26); and
image adjustment interlocking means (46) for interlocking operations of the switching means (22) and the image quality adjustment means (26);
wherein adjusting luminance and chroma suitable for the display image switched by the switching means (22) to be displayed on the monitor (15) are switched synchronized with the switching of signals to be displayed on the monitor (15) by the switching section (22).

2. The ultrasonic diagnostic apparatus according to claim 1, wherein the second region display means (24) reduces the size of the endoscopic optical image to be displayed on a part of the display screen of the monitor (15) and displays the endoscopic optical image.

3. The ultrasonic diagnostic apparatus according to claim 1, wherein the third region display means (25) superimposes the blood flow dynamic state image on the ultrasonic tomographic image displayed on the display screen of the monitor (15).

4. The ultrasonic diagnostic apparatus according to claim 1, comprising display limiting means (22) for not displaying the blood flow dynamic state image by the third region display means (25) if the endoscopic optical image is displayed on the monitor (15) by the first region display means (23) by the switching means (22).

5. The ultrasonic diagnostic apparatus according to claim 1, comprising:
external image input means (53) for inputting the endoscopic optical image as a plurality of video signals;
ultrasonic image input means (32) for inputting the ultrasonic tomographic image and the blood flow dynamic state image as a plurality of video signals;
external video signal selecting means (31) for selecting an arbitrary video signal from the plurality of video signals input by the external image input means (53);
external input video converting means (31) for converting into external video data by using the video signal selected by the external video signal selecting means (31);
internal video signal selecting means (33) for selecting an arbitrary video signal from the plurality of video signals input by the ultrasonic image input means (32) and for converting the video signal selected by the ultrasonic video signal selecting means (32) into internal data;
video size converting means for adjusting the image size of the external video data output by the external video converting means (31);
image combining means (32) for combining the internal video data output by the internal video converting means and the external conversion video data output by the video size converting means; and
video data converting means (33) for converting the combined video data output by the image combining means (32) into a plurality of video signals.

6. The ultrasonic diagnostic apparatus according to claim 1, comprising:
external image input means (34) for inputting the endoscopic optical image;
ultrasonic data input means (35) for inputting the ultrasonic reception data;
external input video converting means (37) for converting from the video signal input by the external image input means into external video data;
external data transmitting means (38) for transmitting the external video data to an image generating unit (36);
ultrasonic tomographic image processing means (39) for generating ultrasonic tomographic image data from the ultrasonic reception data input by the ultrasonic data input means (35) and Doppler processing means (40) for generating blood flow dynamic state data;
internal data transmitting means (41) for transmitting the ultrasonic tomographic image data obtained by the ultrasonic tomographic image processing means (39) and the blood flow dynamic state data obtained by the Doppler processing means to the image generating unit (36);
the image generating unit (36) being provided with data receiving means (42) for receiving data transmitted from the internal data transmitting means (41) and the external data transmitting means (38);
storing means (44) for storing various data received from the data receiving means (38, 41);
processing means (43) for reading out the various data from the storing means (44) and performing an image correction processing; and
image outputting means (45) for converting the image data to which the image correction processing is performed by the image correction processing means into a video signal.

## Patentansprüche

1. Ultraschall-Diagnosegerät, das dazu eingerichtet ist, eine Ultraschallwelle an ein Inneres eines Subjekts zu übertragen, die reflektierte Welle von einem lebenden Körpergewebe zu empfangen, ein tomographisches Ultraschallbild in dem Inneren des Subjekts zu erhalten, während ein endoskopisches optisches Bild in dem Inneren des Subjekts erhalten wird, und das tomografische Ultraschallbild und/oder das endoskopische optische Bild auf einem Monitor (15) anzuzeigen, wobei das Ultraschall-Diagnosegerät umfasst:
ein erstes Bereichsanzeigemittel (23) zum Anzeigen des tomografische Ultraschallbilds oder des endoskopischen optischen Bilds in einem ersten Anzeigebereich (28) des Anzeigebildschirms des Monitors (15);
ein zweites Bereichsanzeigemittel (24) zum Anzeigen des endoskopischen optischen Bilds in einem zweiten Anzeigebereich (29) auf einem Teil des Anzeigebildschirms des Monitors (15);
**dadurch gekennzeichnet, dass**
das Ultraschall-Diagnosegerät ferner dazu eingerichtet ist, ein Bild eines dynamischen Blutflusszustands zu erhalten und anzuzeigen und ferner umfasst:
ein drittes Bereichsanzeigemittel (25) zum Überlagern des Bilds eines dynamischen Blutflusszustands in einem dritten Anzeigebereich (30) auf dem in dem ersten Anzeigebereich (29) auf dem Anzeigebildschirm des Monitors (15) angezeigten tomografischen Ultraschallbild;
ein Schaltmittel (22) zum Umschalten zwischen dem tomografischen Ultraschallbild und dem auf dem Monitor (15) durch das erste Bereichsanzeigemittel (23) während des Umschaltens angezeigten endoskopischen optischen Bild, um das endoskopische optische Bild durch das zweite Bereichsanzeigemittel (24) und/oder das Bild eines dynamischen Blutflusszustands durch das dritte Bereichsanzeigemittel (25) anzuzeigen, wenn das tomografische Ultraschallbild durch das erste Bereichsanzeigemittel (23) angezeigt wird;
ein Bildqualitätseinstellmittel (26) zum Einstellen einer Helligkeit und einer Chroma eines auf dem Monitor (15) durch das erste Bereichsanzeigemittel (23), das zweite Bereichsanzeigemittel (24) bzw. das dritte Bereichsanzeigemittel (25) angezeigten Bilds;
ein Bildqualitätseinstelldatenspeichermittel (52) zum Speichern von Einstellinhalten des Bildqualitätseinstellmittels (26); und
ein Bildeinstellungsverbindungsmittel (46) zum Verbinden von Operationen des Schaltmittels (22) und des Bildqualitätseinstellmittels (26);
wobei Einstellungen einer Helligkeit und einer Chroma, die für das durch das Schaltmittel (22) zur Anzeige auf dem Monitor (15) umgeschaltete Anzeigebild geeignet sind, synchronisiert mit der Schaltung von Signalen geschaltet werden, die durch das Schaltmittel (22) zur Anzeige auf dem Monitor (15) vorgesehen sind.

2. Ultraschall-Diagnosegerät nach Anspruch 1, bei dem das zweite Bereichsanzeigemittel (24) die Größe des auf einem Teil des Anzeigebildschirms des Monitors (15) anzuzeigenden endoskopischen optischen Bilds reduziert und das endoskopische optische Bild anzeigt.

3. Ultraschall-Diagnosegerät nach Anspruch 1, bei dem das dritte Bereichsanzeigemittel (25) das Bild eines dynamischen Blutflusszustands dem auf dem Anzeigebildschirm des Monitors (15) angezeigten tomografischen Ultraschallbild überlagert.

4. Ultraschall-Diagnosegerät nach Anspruch 1, mit einem Anzeigebegrenzungsmittel (22) zum Nicht-Anzeigen des Bilds eines dynamischen Blutflusszustands durch das dritte Bereichsanzeigemittel (25), wenn das endoskopische optische Bild durch das erste Bereichsanzeigemittel (23) durch das Schaltmittel (22) auf dem Monitor (15) angezeigt wird.

5. Ultraschall-Diagnosegerät nach Anspruch 1, das umfasst:
ein externes Bildeingabemittel (53) zum Eingeben des endoskopischen optischen Bilds als eine Mehrzahl von Videosignalen;
ein Ultraschallbildeingabemittel (32) zum Eingeben des tomografischen Ultraschallbilds und des Bilds eines dynamischen Blutflusszustands als eine Mehrzahl von Videosignalen;
ein externes Videosignalauswahlmittel (31) zum Auswählen eines beliebigen Videosignals aus der Mehrzahl von durch das externe Bildeingabemittel (53) eingegebenen Videosignalen;
ein externes Eingabevideoumwandlungsmittel (31) zum Umwandeln in externe Videodaten unter Verwendung des durch das externe Videosignalauswahlmittel (31) ausgewählten Videosignals;
ein internes Videosignalauswahlmittel (33) zum Auswählen eines beliebigen Videosignals aus der Mehrzahl von durch das Ultraschallbildeingabemittel (32) eingegebenen Videosignalen und zum Umwandeln des durch das Ultraschallvideosignalauswahlmittel (32) ausgewählten Videosignals in interne Daten;
ein Videogrößenumwandlungsmittel zum Anpassen der Bildgröße der durch das externe Videoumwandlungsmittel (31) ausgegebenen externen Videodaten;
ein Bildkombinationsmittel (32) zum Kombinieren der durch das interne Videoumwandlungsmittel ausgegebenen internen Videodaten und der durch das Videogrößenumwandlungsmittel ausgegebenen externen Umwandlungsvideodaten;
und
ein Videodatenumwandlungsmittel (33) zum Umwandeln der von dem Bildkombinationsmittel (32) ausgegebenen kombinierten Videodaten in eine Mehrzahl von Videosignalen.

6. Ultraschall-Diagnosegerät nach Anspruch 1, das umfasst:
ein externes Bildeingabemittel (34) zum Eingeben des endoskopischen optischen Bilds;
ein Ultraschalldateneingabemittel (35) zum Eingeben der Ultraschallempfangsdaten;
ein externes Eingabevideoumwandlungsmittel (37) zum Umwandeln von dem von dem externen Bildeingabemittel eingegebenen Videosignal in externe Videodaten;
ein externes Datenübertragungsmittel (38) zum Übertragen der externen Videodaten an eine Bilderzeugungseinheit (36);
ein Mittel (39) zum Verarbeiten eines tomografischen Ultraschallbilds zum Erzeugen von tomografischen Ultraschallbilddaten aus den von dem Ultraschalldateneingabemittel (35) eingegebenen Ultraschallempfangsdaten und ein Doppler-Verarbeitungsmittel (40) zum Erzeugen von Daten über einen dynamischen Blutflusszustand;
ein internes Datenübertragungsmittel (41) zum Übertragen der von dem Mittel (39) zum Verarbeiten eines tomografischen Ultraschallbilds erhaltenen tomografischen Ultraschallbilddaten und der von dem Doppler-Verarbeitungsmittel erhaltenen Daten über einen dynamischen Blutflusszustand an die Bilderzeugungseinheit (36);
wobei die Bilderzeugungseinheit (36) mit einem Datenempfangsmittel (42) zum Empfangen von von dem internen Datenübertragungsmittel (41) und dem externen Datenübertragungsmittel (38) übertragenen Daten versehen ist;
ein Speichermittel (44) zum Speichern von verschiedenen von dem Datenempfangsmittel (38, 41) empfangenen Daten;
ein Verarbeitungsmittel (43) zum Auslesen der verschiedenen Daten aus dem Speichermittel (44) und zum Durchführen einer Bildkorrekturverarbeitung; und
ein Bildausgabemittel (45) zum Umwandeln der Bilddaten, an denen von dem Bildkorrekturverarbeitungsmittel die Bildkorrekturverarbeitung durchgeführt wird, in ein Videosignal.

## Revendications

1. Appareil de diagnostic à ultrasons configuré pour transmettre une onde ultrasonore vers l'intérieur d'un sujet, recevoir l'onde réfléchie depuis un tissu de corps vivant, obtenir une image tomographique ultrasonore de l'intérieur du sujet, tout en obtenant une image optique endoscopique de l'intérieur du sujet, et pour afficher au moins l'une parmi l'image tomographique ultrasonore et l'image optique endoscopique sur un moniteur (15), l'appareil de diagnostic à ultrasons comprenant :
un premier moyen (23) d'affichage de région destiné à afficher l'une parmi l'image tomographique ultrasonore ou l'image optique endoscopique dans une première région d'affichage (28) de l'écran d'affichage du moniteur (15) ;
un deuxième moyen (24) d'affichage de région destiné à afficher l'image optique endoscopique dans une deuxième région d'affichage (29) sur une partie de l'écran d'affichage du moniteur (15) ;
**caractérisé en ce que**
l'appareil de diagnostic à ultrasons est en outre configuré pour obtenir et afficher une image de l'état dynamique du débit sanguin et comprend en outre :
un troisième moyen (25) d'affichage de région destiné à superposer l'image de l'état dynamique du débit sanguin dans une troisième région d'affichage (30) sur l'image tomographique ultrasonore affichée dans la première région d'affichage (29) sur l'écran d'affichage du moniteur (15) ;
un moyen de commutation (22) destiné à commuter entre l'image tomographique ultrasonore et l'image optique endoscopique affichée sur le moniteur (15) par le premier moyen (23) d'affichage de région tout en commutant de façon à afficher l'image optique endoscopique par le deuxième moyen (24) d'affichage de région et/ou l'image de l'état dynamique du débit sanguin par le troisième moyen (25) d'affichage de région lorsque l'image tomographique ultrasonore est affichée par le premier moyen (23) d'affichage de région ;
un moyen (26) d'ajustement de qualité d'image destiné à ajuster la luminance et la saturation des couleurs d'une image affichée sur le moniteur (15) par le premier moyen (23) d'affichage de région, le deuxième moyen (24) d'affichage de région, et le troisième moyen (25) d'affichage de région respectivement ;
un moyen (52) de mémorisation de données d'ajustement de qualité d'image destiné à mémoriser des contenus d'ajustement du moyen (26) d'ajustement de qualité d'image ; et
un moyen (46) de verrouillage d'ajustement d'image destiné à verrouiller les opérations du moyen de commutation (22) et du moyen (26) d'ajustement de qualité d'image ;
dans lequel l'ajustement de la luminance et de la saturation des couleurs appropriées pour l'image d'affichage commutée par le moyen de commutation (22) devant être affichée sur le moniteur (15) sont commutées en synchronisme avec la commutation des signaux devant être affichés sur le moniteur (15) par la section de commutation (22).

2. Appareil de diagnostic à ultrasons selon la revendication 1, dans lequel le deuxième moyen (24) d'affichage de région réduit la taille de l'image optique endoscopique devant être affichée sur une partie de l'écran d'affichage du moniteur (15) et affiche l'image optique endoscopique.

3. Appareil de diagnostic à ultrasons selon la revendication 1, dans lequel le troisième moyen (25) d'affichage de région superpose l'image de l'état dynamique du débit sanguin sur l'image tomographique ultrasonore affichée sur l'écran d'affichage du moniteur (15).

4. Appareil de diagnostic à ultrasons selon la revendication 1, comprenant un moyen (22) de limitation d'affichage destiné à ne pas afficher l'image de l'état dynamique du débit sanguin par le troisième moyen (25) d'affichage de région si l'image optique endoscopique est affichée sur le moniteur (15) par le premier moyen (23) d'affichage de région par le moyen de commutation (22).

5. Appareil de diagnostic à ultrasons selon la revendication 1, comprenant :
un moyen (53) d'entrée d'image externe destiné à délivrer en entrée l'image optique endoscopique comme une pluralité de signaux vidéo ;
un moyen (32) d'entrée d'image ultrasonore destiné à délivrer en entrée l'image tomographique ultrasonore et l'image de l'état dynamique du débit sanguin comme une pluralité de signaux vidéo ;
un moyen (31) de sélection de signal vidéo externe destiné à sélectionner un signal vidéo arbitraire à partir de la pluralité de signaux vidéo délivrés en entrée par le moyen (53) d'entrée d'image externe ;
un moyen (31) de conversion vidéo d'entrée externe destiné à convertir en données vidéo externes en utilisant le signal vidéo sélectionné par le moyen (31) de sélection de signal vidéo externe ;
un moyen (33) de sélection de signal vidéo interne destiné à sélectionner un signal vidéo arbitraire à partir de la pluralité de signaux vidéo délivrés en entrée par le moyen (32) d'entrée d'image ultrasonore et destiné à convertir le signal vidéo sélectionné par le moyen (32) de sélection de signal vidéo ultrasonore en données internes ;
un moyen de conversion de taille de vidéo destiné à ajuster la taille d'image des données vidéo externes délivrées en sortie par le moyen (31) de conversion vidéo externe ;
un moyen (32) de combinaison d'image destiné à combiner les données vidéo internes délivrées en sortie par le moyen de conversion vidéo interne et les données vidéo de conversion externes délivrées en sortie par le moyen de conversion de taille de vidéo ; et
un moyen (33) de conversion de données vidéo destiné à convertir les données vidéo combinées délivrées en sortie par le moyen (32) de combinaison d'image en une pluralité de signaux vidéo.

6. Appareil de diagnostic à ultrasons selon la revendication 1, comprenant :
un moyen (34) d'entrée d'image externe destiné à délivrer en entrée l'image optique endoscopique ;
un moyen (35) d'entrée de données ultrasonores destiné à délivrer en entrée les données de réception ultrasonores ;
un moyen (37) de conversion vidéo d'entrée externe destiné à convertir le signal vidéo délivré en entrée par le moyen d'entrée d'image externe en données vidéo externes ;
un moyen (38) de transmission de données externes destiné à transmettre les données vidéo externes vers une unité (36) de génération d'image ;
un moyen (39) de traitement d'image tomographique ultrasonore destiné à générer des données d'image tomographique ultrasonores à partir des données de réception ultrasonores délivrées en entrée par le moyen (35) d'entrée de données ultrasonores et un moyen (40) de traitement Doppler pour générer des données d'état dynamique du débit sanguin ;
un moyen (41) de transmission de données internes destiné à transmettre les données d'image tomographique ultrasonores obtenues par le moyen (39) de traitement d'image tomographique ultrasonore et les données d'état dynamique du débit sanguin obtenues par le moyen de traitement Doppler vers l'unité (36) de génération d'image ;
l'unité (36) de génération d'image étant munie d'un moyen (42) de réception de données destiné à recevoir les données transmises depuis le moyen (41) de transmission de données internes et le moyen (38) de transmission de données externes ;
un moyen (44) de mémorisation destiné à mémoriser diverses données reçues depuis le moyen (38, 41) de réception de données ;
un moyen (43) de traitement destiné à extraire les diverses données provenant du moyen de mémorisation (44) et à réaliser un traitement de correction d'image ; et
un moyen (45) de sortie d'image destiné à convertir les données d'image sur lesquelles le traitement de correction d'image est exécuté par le moyen de traitement de correction d'image en un signal vidéo.
